# EUROPEAN PATENT APPLICATION

(11) **EP 2 080 527 A1**
(43) Date of publication of application: **22.07.2009**
(21) Application number: 08172639.0
(22) Date of filing: 22.12.2008
(51) Int. Cl.: A61L 2/18

(54) **Process and system for treating moving and stationary parts in uht sterilization plants**

(30) Priority: 21.01.2008 IT PR20080005
(71) Applicant: CFT S.p.A., 43100 Parma (IT)
(72) Inventor: Catelli, Roberto, 43100, PARMA (IT); Lazzari, Alessio, 46019, SALINA DI VIADANA (MANTOVA) (IT)
(74) Representative: Leone, Mario

(57) **Abstract**

A process for treating moving and/or stationary parts in UHT sterilization plants, which includes the steps of mixing an amount of ozone with water to sterilize it and introducing said mixture into the moving and/or stationary parts of UHT sterilization plants; the mixture so obtained retaining its sterilizing power by continuous supply of ozone, for the relevant moving or stationary parts through which it passes. The sterile sterilizing barrier is interposed between the external environment and the internal aseptic circuits of the UHT plant and the moving parts, such as homogenizer pistons, aseptic pumps, seals of centrifugal pumps and static aseptic seals.

## Description

The present invention relates to a process and a system for treating moving and/or stationary parts in UHT sterilization plants, particularly for creating a sterile sterilizing barrier, as well as for lubrication and/or cooling.

The sterile barrier is interposed between the external environment and the internal aseptic circuits of the UHT plant and the moving parts, such as homogenizer pistons, aseptic pumps, seals of centrifugal pumps and static aseptic seals.

The current system for creating the sterile barrier and for lubricating and/or cooling moving parts operates by sterilizing the heat exchanger and the circuit using direct contact steam, condensing steam using the heat exchanger and thus generating sterile condensate at 60/70°C with a cost of about € 0.026 per liter of condensate produced.

Drawbacks of the prior art essentially relate to circuit sterilization and the risk of losing sterility during condensate production, in addition to the high production costs required for generating steam and later cooling it (about € 0.026 per liter of condensate produced, which are required for generating steam and later cooling it).

In another known and implemented method, the sterile (lubricating and/or cooling) barrier is created using cold water sterilized by filtration.

Namely, the implemented process draws well or demineralized water through at least three filters (F1, F2, F3) and then transfers said filtered and sterilized water to the moving parts; sterilization of the filters and the water distribution circuit starts from at least one tank (S) with the addition of an antiseptic and antibacterial agent in proportion to the tank capacity:
the filters (F1, F2, F3) have increasing filtering
grades and at least the last filter (F3) is a
bacteriological filter.

As compared with the above mentioned process, the latter provides a number of improvements to the syste, such as:
➢ Reduction of production costs, thanks to the use of water without having to turn it into steam,
➢ Lower maintenance requirements for plant equipment, thanks to the use of water at a lower temperature, i.e. 14/18°C, wherefore moving part seals exhibit longer lives, even twice as long, and maintenance costs are halved,
➢ Reduced energy consumption in the plant, which results in reduced emission of exhaust gases of steam production boilers to the atmosphere,
➢ Full automation of both the sterilization and production steps.

Nonetheless, this system still suffers from certain drawbacks, namely:
- antibacterial agents and sterilizing solutions are required to be added to the tank,
- the water sterilized in the filtration process creates the barrier but has no sterilizing power.

The object of this invention is to obviate the above drawbacks by using a process and a system for continuously creating a sterile sterilizing barrier and for lubrication and/or cooling in which the sterile battier is created and maintained by the use of water added with ozone, which ozone is dispersed in water using a special generator and a static or dynamic dispersion apparatus or the like, as disclosed and claimed hereinafter.

The following advantages are achieved:
➢ No antibacterial agents or sterilizing solutions are needed
➢ The system has a continuous self-sterilizing power, because the ozone dispersed in water will continuously sterilize water, the circuit and the moving and/or stationary parts
➢ Full automation of both sterilization and production steps
➢ Lower service investment costs in case of addition of a new UHT line, due to lower steam requirements for the UHT plant
➢ Product burn-on at the inner surfaces of both dynamic and static connections is prevented, the connections being maintained in aseptic conditions by cold ozonized sterilizing water.

These objects and advantages are achieved by the process and system for treating moving parts in IHT sterilization plants and for creating a sterile sterilizing barrier and for lubricating and/or cooling moving parts in UHT sterilization plants according to this invention, which is characterized by the annexed claims.

This and other features will be more apparent from the following description of a few variants of a preferred embodiment, which is shown by way of example and without limitation in the accompanying drawings, in which:

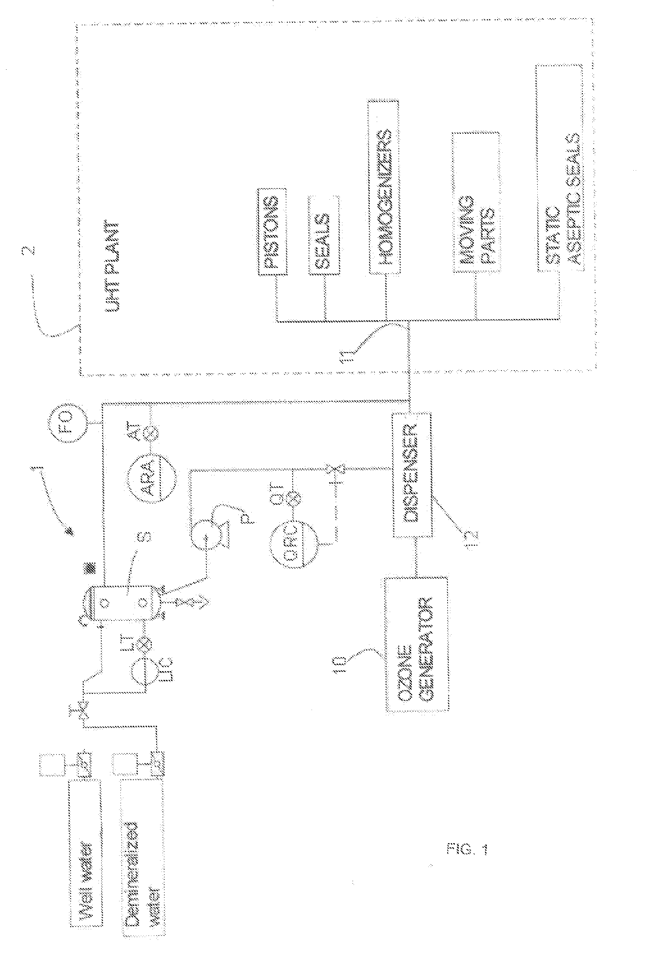

- Figure 1 schematically shows the operation of the system for creating a sterile barrier and lubricating and/or cooling moving and/or stationary parts in UHT sterilization plants according to the present invention.

Referring to Figure 1, numeral 1 generally designates a diagram for the sterilization system and process of the invention and numeral 2 namely designates by a dashed outline the area defined by a possible UHT sterilization plant including many different elements, such as tanks for receiving the product, high and/or low pressure centrifugal pumps for feeding the product, product filters, shell-and-tube exchangers, steam injection sterilizers, holding tanks for maintaining a constant sterilization temperature, vacuum flash coolers for instantaneous cooling, aseptic centrifugal pumps, aseptic homogenizers.

Also, there will be overpressure valves, homogenizing valves, thermistors, pressure gauges, pressure reducers, drives, electric motors and static aseptic seals.

Particularly the plant 2 has, distributed therein, homogenizer pistons, aseptic pumps, mechanical seals of centrifugal and lobed pumps and other moving parts, all requiring a sterile barrier to the outside, lubrication and cooling in a fully aseptic environment

Generally, once the plant 2 has reached the production temperature conditions, the product is fed to a constant level tank and is transferred, by the first centrifugal pump, to a shell-and-tube exchanger where it is heated to about 80/85° using the heat in the vapors extracted from the flash cooler.

From there it flows into a second shell-and-tube stabilizing exchanger, whereupon it flows into the injection head and is instantaneously heated to 150°C by direct steam injection.

After about 6 seconds in the tubular holding tank, whereupon sterilization occurs, the product flows into the flash cooler where instantaneous cooling by expansion, vapor extraction and restoration of the initial solid content, as well as removal of off flavors, occur.

Now, a second centrifugal pump, which also has sterility preserving equipment, feeds the product to the homogenizer to ensure that no phase separation occurs during extended preservation times.

The next steps are final cooling in a shell-and-tube exchanger using water, optional feeding to intermediate aseptic tanks or, at the end of the cycle, directly to aseptic packagers.

The system for creating an aseptic barrier and lubricating as defined hereinbelow can create a sterile barrier and lubricate and/or cool moving parts, such as pistons or centrifugal pumps or seals by only using water added with ozone, which is obtained by an appropriate ozone generator apparatus 10 and a system for ozone dispersion in water.

For instance, a static Venturi mixer or a dynamic mixer may be used as a system for adding ozone into the water conduit.

Water is typically cold, i.e. at source or ambient temperature, and may be natural, softened and/or osmotized.

The ozone delivery unit may be of any type.

Once water has passed through static aseptic seals, conduits and moving parts of the system, it will discharged to the drain.

The system also provides checking that ozone is actually present, using checking means or instruments attached to the connection pipes; if a wrong signal is detected, the operator will receive an appropriate warning; optionally, the plant may be shut.

The amount of ozone produced by the generator 10 and mixed to water in the plant 1 falls in a range of 1 to 8 parts per million.

As mentioned above, the present process also conveniently allows water added with ozone to be transferred to the static aseptic connections of the plant, instead of the conventional steam barrier.

Replacement of steam in the aseptic barriers at static connections, will prevent any product burn-on due to overheating of the aseptic connections, caused by the flow of steam that acts as an aseptic barrier.

With the system as disclosed and claimed herein, no further sterilization will be needed for the circuit and the plant parts to be protected and/or lubricated, because they will be continuously sterilized by water added with ozone.

## Claims

1. A process for treating moving parts in UHT sterilization plants, **characterized in that** it includes the steps of:
a. Mixing an amount of ozone with water; the water so obtained being sterilized with the added ozone,
b. Feeding said water and ozone mixture into the moving parts of UHT sterilization plants; the mixture so obtained having a continuous sterilizing power and being adapted to create a sterile barrier and to lubricate and/or cool said moving parts.

2. A process as claimed in claim 1, **characterized in that** the amount of ozone to be added to water falls in a range from 1 ppm to 6 ppm.

3. A process as claimed in claim 1, **characterized in that** said water is cold, i.e. at source or ambient temperature, softened and/or osmotized.

4. A system as claimed in claims 1 and 2, **characterized in that** it provides checking that ozone is actually present, using downstream checking and recording means or instruments installed on the ozone dispersion system; if a wrong signal is detected, the operator will receive an appropriate warning; optionally, the plant may be shut down.

5. A process as claimed in claim 1, **characterized in that** it includes control of the amount of water and ozone required by the parts to be protected with a sterile sterilizing barrier and for lubrication by a flow rate regulator.

6. A process as claimed in claim 1, **characterized in that** water added with ozone is transferred to the static aseptic connections of the plant, instead of the conventional steam barrier.

7. A process as claimed in claim 1, **characterized in that** replacement of steam in the aseptic barriers at static connections, will prevent any product burn-on due to overheating of the aseptic connections, caused by the flow of steam that acts as an aseptic barrier.
